# NEUE EUROPÄISCHE PATENTSCHRIFT

(11) **EP 0 726 752 B2**
(45) Veröffentlichungstag und Bekanntmachung des Hinweises auf die Entscheidung über den Einspruch: **29.01.2003**
(45) Hinweis auf die Patenterteilung: 14.04.1999
(21) Anmeldenummer: 95900105.8
(22) Anmeldetag: 02.11.1994
(51) Int. Cl.: A61F 13/46, A61F 13/15

(54) **ABSORBIERENDE STRUKTUR**
ABSORBENT STRUCTURE
STRUCTURE ABSORBANTE

(30) Priorität: 10.11.1993 DE 4338326
(43) Veröffentlichungstag der Anmeldung: 21.08.1996
(73) Patentinhaber: Paul Hartmann Aktiengesellschaft, 89522 Heidenheim (DE)
(72) Erfinder: HERMANN, Klaus, D-89537 Giengen (DE); MALOWANIEC, Krzysztof, D., D-89522 Heidenheim (DE); WURSTER, Thomas, D-89522 Heidenheim (DE)
(74) Vertreter: Dreiss, Fuhlendorf, Steimle & Becker
(86) Internationale Anmeldenummer: EP9403602
(87) Internationale Veröffentlichungsnummer: WO95013042

(56) Entgegenhaltungen:
- EP-A- 0 198 683
- EP-A- 0 343 941
- EP-A- 0 478 011
- EP-A- 0 525 778
- WO-A-91/10416
- WO-A-91/11162
- WO-A-91/11163
- WO-A-92/11831
- WO-A-92/14430
- WO-A-93/15702
- CA-A- 806 352
- GB-A- 2 266 464
- US-A- 4 578 068

## Beschreibung

Die Erfindung betrifft eine absorbierende Struktur für ein Hygieneprodukt, wie beispielsweise eine Windel, Damenbinde oder Inkontinenzprodukt oder für ein medizinisches Produkt, wie Verbandstoff, gemäß dem Oberbegriff des Anspruchs 1.

Aus der WO 91/11162 ist eine zweilagige absorbierende Struktur für eine Wegwerfwindel bekannt. Die den Saugkörper der Windel bildende absorbierende Struktur besteht aus zwei übereinander gelegten Lagen, wobei die obere, dem Körper zugewandte Lage die anfallende Flüssigkeit aufnimmt und verteilt und die darunter liegende Lage die Flüssigkeit speichert. Die Flüssigkeitsspeicherschicht weist einen hohen Anteil an superabsorbierendem Material auf, wohingegen die Verteilerschicht im wesentlichen frei von superabsorbierendem Material ist. Die Verteilerschicht besteht aus Zellstoff-Fasern, die mittels eines Vernetzungsmittels chemisch versteift sind, in der Weise, daß im wesentlichen Vernetzungen nur innerhalb einer Faser und nicht zwischen den Fasern auftreten.

Nach mehrfachem Flüssigkeitsanfall kann die Speicherschicht aufgrund des hohen Anteils an superabsorbierendem Material keine Flüssigkeit mehr in dem Bereich, wo die Flüssigkeit angefallen ist, aufnehmen, so daß die Flüssigkeit in der Verteilerschicht verbleibt. Die Verteilerschicht nimmt die Flüssigkeit im wesentlichen mittels zwischen den Fasern gebildeten Räumen auf, so daß die Flüssigkeit aus der Verteilerschicht unter leichten Druck austritt. Dadurch ist der Körper eines Trägers der Windel in Kontakt mit der Flüssigkeit, die von der Windel aufgenommen werden sollte.

Ein weiterer Nachteil der bekannten Windel besteht darin, daß das in der Speicherschicht in großer Konzentration auftretende superabsorbierende Material, das in der Regel in körniger Form vorliegt, beim Komprimieren der Windel im Herstellungsprozess die an die Speicherschicht angrenzende Folie verprägt oder gar perforiert.

Aus der WO-A 91/10416 ist eine mehrlagige Saugkörperstruktur bekannt, bei der eine obere, dem Körper zugewandte Lage zumindest eine verdichtete Verteilerschicht und eine weniger verdichtete, superabsorbierendes Material enthaltende Speicherschicht aufweist. Auf der körperabgewandten Seite der oberen Lage ist eine untere Lage aus Zellstoff-Fasern vorgesehen. Mit einer derartigen Saugkörperstruktur soll das superabsorbierende Material effizienter nutzbar sein.

Aus der WO 91/10 416 ist ferner eine sehr vielschichtige Saugkörperstruktur bekannt, die von der körperzugewandten Seite ausgehend eine aus hydrophilen Stapelpolyesterfasern bestehende Schicht, eine verdichtete hydrophile Zellulosefaserschicht, eine Schicht aus hydrophoben thermoplastischen Fasern, eine weitere Schicht aus verdichteten hydrophilen Zellulosefasern und darunter eine Schicht aus hydrophilen oder hydrophoben Fasern oder eine Schaumstruktur aufweist. Dieser Aufbau erweist sich als äußerst aufwendig.

Die WO 93/15 702, die eine Saugkörperstruktur nach dem Oberbegriff des Anspruchs 1 zeigt, lehrt, die Schichten so aufzubauen, dass sich ein bestimmtes Porenvolumen ergibt, um die Flüssigkeitsaufnahmefähigkeit und die Fähigkeit zur Flüssigkeitsverteilung innerhalb der Schichten einstellen zu können.

Ausgehend von diesem Stand der Technik ist es Aufgabe der Erfindung, eine verbesserte absorbierende Struktur bereitzustellen, die die anfallende Flüssigkeit besser aufnimmt und verteilt.

Diese Aufgabe wird gelöst durch einen Gegenstand mit den Merkmalen des Patentanspruchs 1.

Erfindungsgemäß weisen die obere und untere Schicht sowie die untere Lage Zellstoff-Fasern mit unterschiedlicher Absorptionsfähigkeit auf, wobei die Absorptionsfähigkeit der Zellstoff-Fasern der oberen Schicht deutlich geringer ist als die der Zellstoff-Fasern der unteren Schicht und deutlich geringer als die der Zellstoff-Fasern der unteren Lage. Die Zellstoff-Fasern der oberen Schicht sind vernetzte Zellstoff-Fasern und die der unteren Schicht sind nicht vernetzte Zellstoff-Fasern.

Insgesamt kann die absorbierende Struktur mehrere Flüssigkeitsanfälle besser aufnehmen.

Ein weiterer Vorteil der unteren Lage besteht darin, daß diese kein oder nur wenig superabsorbierendes Material, das in der Regel in Körnerform vorliegt, aufweist. Dadurch wird eine an der unteren Lage anliegende Folie bei der Komprimierung der absorbierenden Struktur in der Produktion nicht verprägt oder gar beschädigt. Desweiteren gibt die aus Zellstoff-Fasern bestehende untere Lage der absorbierenden Struktur eine angenehmere Weichheit.

Vorteilhafte Ausgestaltungen sind Gegenstand der Unteransprüche.

In der Ausgestaltung gemäß Anspruch 2 sind die Fasern der unteren Lage dünner, wodurch bei gleicher Dichte eine größere Anzahl Fasern pro Volumen vorliegt und damit eine größere Anzahl von Kapillaren. Dadurch wird weitere Flüssigkeit aus der Speicherschicht in die untere Lage gesogen.

Besonders bevorzugte Ausgestaltungen sind in den Ansprüchen 3 bis 12 beschrieben. So ist vorgesehen, daß die beiden Schichten der oberen Lage, Verteilerschicht und Speicherschicht, nicht einfach übereinander liegen, sondern innig miteinander verbunden sind, so daß zwischen den beiden Schichten eine Übergangszone gebildet ist, in der die Komponenten der beiden Schichten gemischt vorliegen. In dieser Übergangszone liegt ein Übergang von einer Faserart zur anderen vor, gemäß den Ansprüchen 4 und 5. Dadurch kann die anfallende Flüssigkeit besser von der oberen Schicht, also der Verteilerschicht in die untere Schicht, also der Speicherschicht, geleitet werden. Die Verteilerschicht ist schneller für den nächsten Flüssigkeitsanfall bereit. Der Übergang von einer Faserart zur anderen ist entweder kontinuierlich, also graduell oder diskontinuierlich, beispielsweise in mehreren Stufen.

Die Konzentration des in der Speicherschicht vorliegenden superabsorbierenden Materials nimmt in der Übergangszone in Richtung der Verteilerschicht kontinuierlich oder diskontinuierlich ab. Die Konzentrationsabnahme kann gemäß Anspruch 7 sich über die gesamte Dicke der unteren Schicht hinziehen. Aufgrund des Konzentrationsgradienten kann die Flüssigkeit besser an unverbrauchte, also trockene Stellen der absorbierenden Struktur geleitet werden.

Für die obere Schicht der oberen Lage sind vorteilhafterweise intrafaservernetzte Zellstoff-Fasern vorgesehen. Derartige Fasern nehmen kaum Flüssigkeit auf und quellen nicht, so daß die Flüssigkeit problemlos von der darunter liegenden Speicherschicht aufgenommen werden kann. Die obere Schicht aus vernetzten Zellstoff-Fasern fällt bei Befeuchtung nicht zusammen, so daß weitere Flüssigkeitsanfälle von der Verteilerschicht aufgenommen werden können.

Die untere Lage weist bevorzugt 0 bis maximal 10 Gew.% an superabsorbierendem Material auf und die Speicherschicht etwa 10 bis 98 Gew.%.

Die Erfindung wird im folgenden anhand eines Ausführungsbeispiels unter Bezugnahme auf die Zeichnung im einzelnen beschrieben. In der Zeichnung zeigen:
- Figur 1: einen Längsschnitt durch eine Windel, die die erfindungsgemä8e, absorbierende Struktur aufweist,
- Figur 2: eine Draufsicht auf die absorbierende Struktur aus Figur 1.

In dem in der Zeichnung dargestellten Ausführungsbeispiel bildet eine absorbierende Struktur 10 einen Saugkörper 16 einer Windel 11. Die Windel 11 weist eine Wäscheschutzfolie 12, eine flüssigkeitsdurchlässige und dem Körper zugewandte Vliesstoffabdeckung 14 sowie den zwischen Wäscheschutzfolie 12 und Vliesstoffabdeckung 14 angeordneten Saugkörper 16 auf.

Der Saugkörper 16 weist eine obere, dem Körper zugewandte Lage 18 und eine darunter liegende, untere Lage 22 auf, die an einer Grenzfläche 20 übereinander liegen. Die obere Lage 18 weist eine obere, dem Körper zugewandte Schicht 26 und eine untere Schicht 28 auf.

Die obere Schicht 26 besteht aus vernetzten Zellstoff-Fasern, wie sie beispielsweise aus der EP 0 252 650 B1 bekannt sind. Derartige Zellstoff-Fasern speichern die Flüssigkeit nicht, so daß die obere Schicht 26 die Flüssigkeit im wesentlichen über durch die Fasern gebildete Kapillaren aufnimmt und innerhalb der Schicht 26 verteilt. Die obere Schicht 26 sei deshalb im folgenden auch als Verteilerschicht 26 bezeichnet.

Die untere Schicht 28 weist herkömmliche nicht vernetzte Zellstoff-Fasern auf, die eine deutlich höhere Absorptionsfähigkeit haben als vernetzte Fasern und ein superabsörbierendes Material, das beispielsweise in Form von Körnern 29 vorliegt. Der Anteil an superabsorbierendem Material beträgt etwa 10 bis 98 Gew.% und vorzugsweise 30 bis 50 Gew.%. Die Schicht 28 hat die Funktion einer Flüssigkeitsspeicherschicht und sei im folgenden auch als Speicherschicht 28 bezeichnet.

Zwischen der Verteilerschicht 26 und der Speicherschicht 28 befindet sich eine Übergangszone 30, die in der Zeichnug durch Schlangenlinien, die obere und untere Grenzflächen 32 und 34 der Übergangszone 30 andeuten, dargestellt ist.

Innerhalb der Übergangszone 30 nimmt die Dichte der vernetzten Fasern der Verteilerschicht von der oberen Grenzfläche 32 zur unteren Grenzfläche 34 kontinuierlich ab. Andererseits nimmt die Dichte der Fasern der Speicherschicht 28 in gleicher Richtung kontinuierlich zu, so daß in entgegengesetzte Richtungen weisende Dichtegradienten der beiden Faserarten in der Übergangszone 30 vorliegen. In der Übergangszone 30 findet also in Dickenrichtung des Saugkörpers ein gradueller Übergang von einer Faserart zur anderen statt.

Auch das in der Speicherschicht 28 befindliche superabsorbierende Material 29 nimmt in der Übergangszone 30 von der oberen Grenzfläche 32 zur unteren Grenzfläche 34 kontinuierlich zu. Insgesamt sind in der Übergangszone 30 die einzelnen Komponenten der Verteiler- und Speicherschicht 26 und 28 vermischt, so daß ein kontinuierlicher Übergang von der einen zur anderen Schicht vorliegt.

Weiterhin ist vorgesehen, daß die Konzentration des superabsorbierenden Materials der Speicherschicht von der zwischen der oberen und der unteren Lage 18 und 22 liegenden Grenzfläche 20 in Richtung der Verteilerschicht 26 bis zur oberen Grenzfläche 32 der Übergangszone 30 kontinuierlich abnimmt. Über der gesamten Dicke der Speicherschicht 28 liegt somit ein Konzentrationsgradient des superabsorbierenden Materials vor.

In einer weiteren, nicht dargestellten Ausführungsform ist die Änderung der Dichte der Zellstoff-Fasern und/oder die Änderung der Konzentration des superabsorbierenden Materials diskontinuierlich. Dies kann eine stufenweise Änderung sein oder eine Änderung der Dichte- bzw. Konzentrationsgradienten.

Die Speicherschicht 28 liegt auf der unteren Lage 22 auf. Die untere Lage 22 besteht im wesentlichen nur aus nicht vernetzten Zellstoff-Fasern und enthält kein oder nur sehr wenig superabsorbierendes Material, vorzugsweise 0 bis 10 Gew.-%.

In einer bevorzugten Ausgestaltung haben die Zellstoff-Fasern der unteren Lage 22 einen kleineren Querschnitt, sind also dünner, als die Fasern der Speicherschicht 28. Dadurch wird die Anzahl der durch die Fasern gebildeten Kapillaren erhöht, da dünnere Fasern bei gleicher Dichte der Schicht eine größere Anzahl Fasern pro Volumen ergeben. Eine größere Anzahl von Kapillaren in der unteren Lage 22 verbessert die Fähigkeit der unteren Lage 22, Flüssigkeit, die von der Speicherschicht 28 nicht mehr aufgenommen werden kann, aufzunehmen und in andere Bereiche des Saugkörpers 26 zu transportieren.

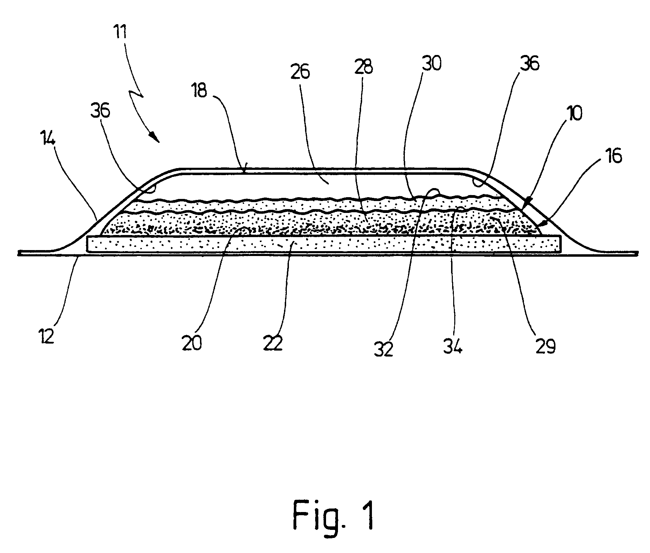

Wie aus der Schnittdarstellung der Fig. 1 hervorgeht, weist die obere Lage 18 abgeschrägt Ränder 36 auf, so daß der Träger der Windel die Ränder 36 kaum spüren wird.

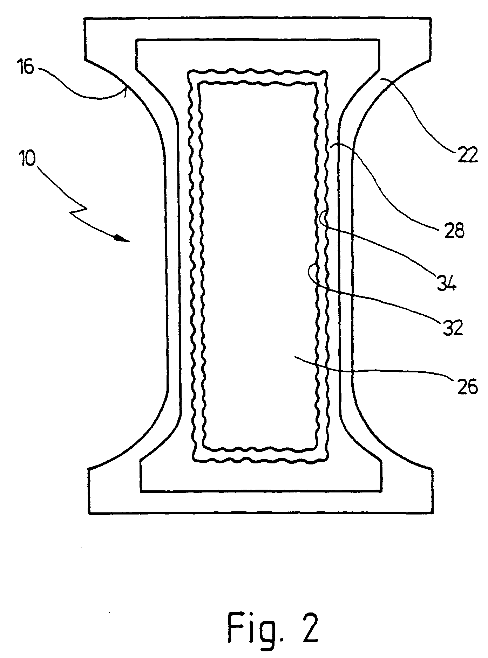

Fig. 2 zeigt die den Saugkörper 16 bildende absorbierende Struktur 10 des bevorzugten Ausführungsbeispiels in einer Draufsicht. Die untere Lage 22 hat einen sanduhrförmigen Zuschnitt wie auch die daraufliegende Speicherschicht 28, deren Längen- und Breitenabmessungen etwas kleiner sind. Die Verteilerschicht 26 hat einen rechteckigen Zuschnitt mit wiederum kleineren Abmessungen als die der Speicherschicht 28. Die Zuxhnitte der einzelnen Lagen und Schichten 22, 25 und 28 können aber auch verschiedenste Zuschnittsformen haben, beispielsweise, sanduhrförmig, rechteckig, oval, rund oder dergleichen. Auch kann vorgesehen sein, daß sich einzelne Lagen oder Schichten nur über Teilbereiche der Windel erstrecken.

Jede Lage 18, 22 bzw. Schicht 26, 28 kann Zusätze, wie superabsorbierendes Material, Bindemittel, Kunststoff-Fasern, Schmelzfasern oder Bikomponentfasern enthalten, obschon die oben beschriebene Zusammensetzung der Lagen bzw. Schichten bevorzugt ist. Bindemittel geben beispielsweise der Lage oder Schicht, der sie zugesetzt werden, eine größere Festigkeit.

## Patentansprüche

1. Absorbierende Struktur für eine Hygieneprodukt, wie Windel, Damenbinde oder Inkontinenzprodukt oder für ein medizinisches Produkt, wie Verbandstoff, mit einer oberen, dem Körper zugewandten Lage (18), die eine obere, dem Körper zugewandte Schicht (26) und eine untere, superabsorbierendes Material enthaltende Schicht (28) aufweist und mit einer darunter liegenden, im wesentlichen nur aus Zellstoff-Fasern bestehenden unteren Lage (22), **dadurch gekennzeichnet, dass** die Absorptionsfähigkeit von Zellstoff-Fasern der oberen Schicht (26) deutlich geringer ist als die von Zellstoff-Fasern der unteren Schicht (28) und deutlich geringer als die von Zellstoff-Fasern der unteren Lage (22) und dass die Zellstoff-Fasern der oberen Schicht (26) vernetzte Zellstoff-Fasern sind und die Zellstoff-Fasern der unteren Schicht (28) nicht vernetzte Zellstoff-Fasern sind.

2. Absorbierende Struktur nach Anspruch 1, **dadurch gekennzeichnet, daß** die Fasern der unteren Lage (22) einen kleineren Querschnitt haben als die Fasern der unteren Schicht (28) der oberen Lage (18).

3. Absorbierende Struktur nach Anspruch 1 oder 2, **dadurch gekennzeichnet, dass** zwischen den beiden Schichten (26 und 28) der oberen Lage (18) eine Übergangszone (30) gebildet ist, in der die Fasern beider Schichten (26 und 28) und das superabsorbierende Material (29) in einer Mischung vorliegen.

4. Absorbierende Struktur nach Anspruch 3, **dadurch gekennzeichnet, dass** die Dichte der Zellstoff-Fasern der oberen Schicht (26) in der Übergangszone (30) in Richtung der unteren Schicht (28) abnimmt.

5. Absorbierende Struktur nach einem der Ansprüche 3 oder 4, **dadurch gekennzeichnet, dass** die Dichte der Zellstoff-Fasern der unteren Schicht (28) in der Übergangszone (30) in Richtung der oberen Schicht (26) abnimmt.

6. Absorbierende Struktur nach Anspruch 3, 4 oder 5, **dadurch gekennzeichnet, daß** die Konzentration des superabsorbierenden Materials (29) der unteren Schicht (28) in der Übergangszone (30) in Richtung der oberen Schicht (26) abnimmt.

7. Absorbierende Struktur nach einem der Ansprüche 3 bis 6, **dadurch gekennzeichnet, daß** die Konzentration des superabsorbierenden Materials (29) der unteren Schicht (28) innerhalb der unteren Schicht (28) von der unteren Lage (22) in Richtung der oberen Schicht (26) abnimmt.

8. Absorbierende Struktur nach einem der Ansprüche 4 bis 7, **dadurch gekennzeichnet, daß** die Änderung der Dichte der Zellstoff-Fasern kontinuierlich ist.

9. Absorbierende Struktur nach einem der Ansprüche 4 bis 7, **dadurch gekennzeichnet, daß** die Änderung der Dichte der Zellstoff-Fasern diskontinuierlich ist.

10. Absorbierende Struktur nach einem der vorhergehenden Ansprüche 4 bis 9, **dadurch gekennzeichnet, daß** die Änderung der Konzentration des superabsorbierenden Materials kontinuierlich ist.

11. Absorbierende Struktur nach einem der vorhergehenden Ansprüche 4 bis 9, **dadurch gekennzeichnet, daß** die Änderung der Konzentration des superabsorbierenden Materials diskontinuierlich ist.

12. Absorbierende Struktur nach einem der vorhergehenden Ansprüche, **dadurch gekennzeichnet, daß** jede Lage (18, 22) der absorbierenden Struktur Zusätze, wie superabsorbierendes Material, Bindemittel, Kunststoff-Fasern, Schmelzfasern oder Bikomponentfasern enthalten kann.

13. Absorbierende Struktur nach einem der vorhergehenden Ansprüche, **dadurch gekennzeichnet, daß** die untere Lage (22) einen Anteil von 0 bis 10 Gew.% an superabsorbierendem Material enthält.

14. Absorbierende Struktur nach einem der vorhergehenden Ansprüche, **dadurch gekennzeichnet, daß** die untere Schicht (28) der oberen Lage (18) einen Anteil von 10 bis 98 Gew.%, vorzugsweise 30 bis 50 Gew.-%, an superabsorbierendem Material (29) enthält.

## Claims

1. An absorbent structure for a hygiene product, such as a nappy, sanitary towel or incontinence product or for a medical product, such as a dressing material, having an upper ply (18) facing towards the body, which ply has an upper layer (26) facing towards the body and a lower layer (28) containing super-absorbent material, and having an underlying lower ply (22) comprising substantially solely cellulose fibres, **characterised in that** the absorbency of cellulose fibres of the upper layer (26) is distinctly less than that of cellulose fibres of the lower layer (28) and distinctly less than that of cellulose fibres of the lower ply (22), and **in that** the cellulose fibres of the upper layer (26) are crosslinked cellulose fibres and the cellulose fibres of the lower layer (28) are uncrosslinked cellulose fibres.

2. An absorbent structure according to claim 1, **characterised in that** the fibres of the lower ply (22) have a smaller cross-section than the fibres of the lower layer (28) of the upper ply (18).

3. An absorbent structure according to claim 1 or 2, **characterised in that** a transition zone (30) is formed between the two layers (26 and 28) of the upper ply (18), in which zone the fibres of both layers (26 and 28) and the super-absorbent material (29) are present as a mixture.

4. An absorbent structure according to claim 3, **characterised in that** the density of the cellulose fibres of the upper layer (26) decreases in the transition zone (30) in the direction of the lower layer (28).

5. An absorbent structure according to one of claims 3 or 4, **characterised in that** the density of the cellulose fibres of the lower layer (28) decreases in the transition zone (30) in the direction of the upper layer (26).

6. An absorbent structure according to claim 3, 4 or 5, **characterised in that** the concentration of the super-absorbent material (29) of the lower layer (28) decreases in the transition zone (30) in the direction of the upper layer (26).

7. An absorbent structure according to one of claims 3 to 6, **characterised in that** the concentration of the super-absorbent material (29) of the lower layer (28) decreases within the lower layer (28) from the lower ply (22) in the direction of the upper layer (26).

8. An absorbent structure according to one of claims 4 to 7, **characterised in that** that the change in density of the cellulose fibres is continuous.

9. An absorbent structure according to one of claims 4 to 7, **characterised in that** the change in density of the cellulose fibres is discontinuous.

10. An absorbent structure according to one of the preceding claims 4 to 9, **characterised in that** the change in concentration of the super-absorbent material is continuous.

11. An absorbent structure according to one of the preceding claims 4 to 9, **characterised in that** the change in concentration of the super-absorbent material is discontinuous.

12. An absorbent structure according to one of the preceding claims, **characterised in that** each ply (18, 22) of the absorbent structure may contain additives, such as super-absorbent material, binder, plastics fibres, fusible fibres or bicomponent fibres.

13. An absorbent structure according to one of the preceding claims, **characterised in that** the lower ply (22) contains a proportion of 0 to 10% by weight of super-absorbent material.

14. An absorbent structure according to one of the preceding claims, **characterised in that** the lower layer (28) of the upper ply (18) contains a proportion of 10 to 98% by weight, preferably of 30 to 50% by weight, of super-absorbent material (29).

## Revendications

1. Structure absorbante pour un produit d'hygiène, tel que des couches, des serviettes hygiéniques, des produits pour incontinents, ou pour un produit médical, tel des pansements, comportant une feuille supérieure (18) qui est orientée vers le corps et qui comporte une couche supérieure (26) orientée vers le corps et une couche inférieure (28) contenant une matière superabsorbante, et une feuille inférieure (22) disposée en dessous et essentiellement constituée de fibres de cellulose, **caractérisée en ce que** la capacité d'absorption des fibres de cellulose de la couche supérieure (26) est nettement plus faible que celle des fibres de cellulose de la couche inférieure (28) et nettement plus faible que celle des fibres de cellulose de la feuille inférieure (22) et **en ce que** les fibres de cellulose de la couche supérieure (26) sont des fibres de cellulose réticulées et les fibres de cellulose de la couche inférieure (28) des fibres de cellulose non réticulées.

2. Structure absorbante selon la revendication 1, **caractérisée en ce que** les fibres de la feuille inférieure (22) ont une coupe transversale plus petite que les fibres de la couche inférieure (28) de la feuille supérieure (18).

3. Structure absorbante selon la revendication 1 ou 2, **caractérisée en ce que**, entre les deux couches (26 et 28) de la feuille supérieure (18) est formée une zone de transition (30) dans laquelle les fibres des deux couches (26 et 28) et la matière superabsorbante (29) forment un mélange.

4. Structure absorbante selon la revendication 3, **caractérisée en ce que** l'épaisseur des fibres de cellulose de la couche supérieure (26) diminue dans la zone de transition (30) en direction de la couche inférieure (28).

5. Structure absorbante selon l'une des revendications 3 ou 4, **caractérisée en ce que** l'épaisseur de des fibres de cellulose de la couche inférieure (28) diminue dans la zone de transition (30) en direction de la couche supérieure (26).

6. Structure absorbante selon l'une des revendications 3, 4 ou 5, **caractérisée en ce que** la concentration de la matière superabsorbante (29) de la couche inférieure (28) diminue dans la zone de transition (30) en direction de la couche supérieure (26).

7. Structure absorbante selon l'une des revendications 3 à 6, **caractérisée en ce que** la concentration de la matière superabsorbante (29) de la couche inférieure (28) diminue à l'intérieur de la couche inférieure (28) en partant de la feuille inférieure (22) en direction de la couche supérieure (26).

8. Structure absorbante selon l'une des revendications 4 à 7, **caractérisée en ce que** la variation de l'épaisseur des fibres de cellulose est continue.

9. Structure absorbante selon l'une des revendications 4 à 7, **caractérisée en ce que** la variation de l'épaisseur des fibres de cellulose est discontinue.

10. Structure absorbante selon l'une des revendications précédentes 4 à 9, **caractérisée en ce que** la variation de la concentration de la matière superabsorbante est continue.

11. Structure absorbante selon l'une des revendications précédentes 4 à 9, **caractérisée en ce que** la variation de la concentration de la matière superabsorbante est discontinue.

12. Structure absorbante selon l'une des revendications précédentes, **caractérisée en ce que** chaque feuille (18, 22) de la structure peut contenir des additifs, tels que de la matière superabsorbante, des liants, des fibres de matière synthétique, des fibres fondues ou des fibres à deux composants.

13. Structure absorbante selon l'une des revendications précédentes, **caractérisée en ce que** la feuille inférieure (22) contient une proportion comprise entre 0 et 10 % en poids de matière superabsorbante.

14. Structure absorbante selon l'une des revendications précédentes, **caractérisée en ce que** la couche inférieure (28) de la feuille supérieure (18) contient une proportion comprise entre 10 et 98 % en poids, de préférence entre 30 et 90 % en poids, de matière superabsorbante (29)
